# EUROPÄISCHE PATENTSCHRIFT

(11) **EP 0 928 183 B1**
(45) Veröffentlichungstag und Bekanntmachung des Hinweises auf die Patenterteilung: **05.12.2001**
(21) Anmeldenummer: 97910294.4
(22) Anmeldetag: 16.09.1997
(51) Int. Cl.: A61K 7/06, A61K 7/48

(54) **VERWENDUNG VON 1-HYDROXY-2-PYRIDONEN ZUR BEHANDLUNG DER SEBORRHOISCHEN DERMATITIS**
USE OF 1-HYDROXY-2-PYRIDONE FOR THE TREATMENT OF SEBORRHEIC DERMATITIS
UTILISATION DE 1-HYDROXY-2-PYRIDONE POUR LE TRAITEMENT DE L'ECZEMA SEBORRHEIQUE

(30) Priorität: 27.09.1996 DE 19639818
(43) Veröffentlichungstag der Anmeldung: 14.07.1999
(73) Patentinhaber: Aventis Pharma Deutschland GmbH, 65929 Frankfurt am Main (DE)
(72) Erfinder: BOHN, Manfred, D-65719 Hofheim (DE); KRAEMER, Karl, Theodor, D-63225 Langen (DE); MARKUS, Astrid, D-65835 Liederbach (DE)
(86) Internationale Anmeldenummer: EP9705070
(87) Internationale Veröffentlichungsnummer: WO9813009

(56) Entgegenhaltungen:
- EP-A- 0 646 369
- WO-A-94/05256
- WO-A-96/02226
- US-A- 5 132 107
- HANEL, H. : "Therapie des seborrhoischen Ekzems mit einem antiphlogistisch wirksamen Antimicotikum" MYCOSES, Bd. 34, Nr. (suppl. 1), 1991, Seiten 91-93, XP002059046
- GROSSHANS, E.; BRESSIEUX, A.: "L' Eczéma Séborrhéique (La Pityrosporose) " ANN. DERMATOL. VENEREOL., Bd. 115, Nr. 1, 1988, Seiten 79-86, XP002059111
- SHAPIRO, J.; MADDIN, S. : "Medicated Shampoos" CLINICS IN DERMATOLOGY, Bd. 14, Nr. 1, Februar 1996, Seiten 123-128, XP002059047
- ALY, R.; BERGER, T.: "Common Superficial Fungal Infections in Patients with AIDS" CLINICAL INFECTIOUS DISEASES, Bd. 22 (suppl. 2), Mai 1996, Seiten 128-132, XP002059048

## Beschreibung

Unter Seborrhoischer Dermatitis wird eine Erkrankung der Kopfhaut verstanden, die sich von einfachen Kopfschuppen durch das Vorhandensein eines Erythems als Zeichen der Entzündung, dem stärkeren Schuppungsgrad mit gelegentlichem Jucken und Brennen sowie durch das Vorkommen ekzematöser Veränderungen an anderen Körperstellen unterscheidet. Sie kann fleckförmig auftreten, befällt aber auch häufiger die ganze Kopfhaut und bezieht oft über den Haaransatz hinaus die Stirn, zirkular den Hals und die Ohren ein. Die Kopfhaut kann in schweren Fällen sekundär infiziert werden, und die Veränderungen können dann eine schwammige Konsistenz, Bläschen- und Krustenbildung zeigen und nässen.

Eine Seborrhoische Dermatitis tritt häufig schon im Säuglingsalter auf und remittiert gewöhnlich spontan im Alter von 8 - 12 Monaten. Die bei Kleinkindern aus Erythem, Schuppung und zuweilen Bläschen und Krusten bestehenden Kopfhautveränderungen können sich spontan innerhalb einiger Wochen zurückbilden, intermittierend wiederauftreten oder während der ganzen Kindheit persistieren. Sie sind häufig mit einem ähnlichen Prozeß um Augenlider, Nase und Ohren herum kombiniert. Später kommt das Leiden gewöhnlich nach der Pubertät vor und kann das ganze Leben bestehen bleiben oder auch an Stärke zunehmen. Ungefähr 1 - 3 % der Bevölkerung sind von dieser Krankheit betroffen.

Es ist bekannt, daß 1-Hydroxy-2-pyridone und deren Salze gegen normale Kopfschuppen, die durch eine klinisch nicht entzündliche - nahezu bei allen Menschen vorkommende - Abschuppung der Kopfhaut charakterisiert sind, Wirksamkeit zeigen (DE 22 34 009). Dokument WO-A-96/02226 beschreibt ein Shampoo zur Behandlung der seborrhoischen Dermatitis enthaltend 1-Hydroxy-2-pyridon, wobei der pH-Wert der Shampoo-Zubeseitung auf pH 7-9 eingestellt wird.

Die aussichtsreichste Behandlungsart der Seborrhoischen Dermatitis war bislang die topische Applikation von Kortikosteroidpräparaten, in jüngerer Zeit hat jedoch die topische Therapie mit antimykotisch wirksamen Substanzen an Bedeutung gewonnen.

Während Kortikosteroidpräparate ihre Wirksamkeit ausschließlich über eine Beeinflussung des Entzündungsprozesses entfalten, sind die antimykotischen Substanzen wie Ketokonazol, ausschließlich gegen die als Verursacher der Seborrhoischen Dermatitis angenommen Hefepilze vom Stamme Pityrosporum wirksam. Die erfindungsgemäßen 1-Hydroxy-2-pyridone vereinigen dagegen die Eigenschaften beider Stoffklassen in einer Substanz und zeigen sowohl antiinflammatorische Wirkung als auch antimykotische Wirksamkeit gegenüber Pityrosporum Stämmen.

Die erfindungsgemäß verwendeten Substanzen reichern sich im Vergleich zu Ketokonazol - selbst nach nur kurzer topischer Kontaktzeit - schnell in die für das Pilzwachstum relevanten Hautschichten an und tragen damit zu einer raschen Heilung bei.

Während Ketokonazol gegenüber Gram-positiven Bakterien in vitro inaktiv ist (Kinsman et al., J. Med. Microbiol (1983) 16, Nr. 2, IV), zeigen die erfindungsgemäß verwendeten Hydroxypyridone Wirksamkeit gegenüber Gram-positiven und Gram-negativen aeroben und anaeroben Bakterien (Dittmar et al., Arzneim.- Forschung, (1981) 31 (II), Nr. 8a, S. 1317 - 1322). Dies ist im Hinblick auf die Behandlung von sekundär infizierten Fällen ein außerordentlich wichtiger Befund.

Die erfindungsgemäß verwendeten Verbindungen haben gegenüber Ketokonazol weiterhin ganz entscheidende Vorteile hinsichtlich ihrer Verarbeitungsmöglichkeiten in pharmazeutischen Zubereitungen. Aufgrund ihrer Löslichkeit in Wasser, Alkoholen und wäßrig alkoholischen Lösungen ist die Herstellung von Haarwässern und transparenten Gelzubereitungen problemlos möglich.

Die erfindungsgemäß verwendeten Zubereitungen können auch zur Behandlung der Pityriasis versicolor, einer oberflächlichen, nicht entzündlichen Hautpilzerkrankung am Stamm, eingesetzt werden.

Die Erfindung betrifft daher die Verwendung von 1-Hydroxy-2-pyridonen der Formel I, 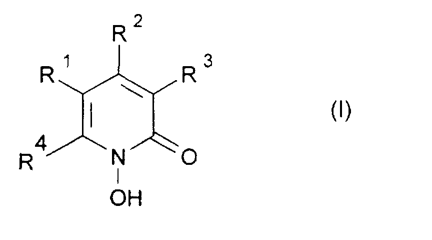 worin
R¹, R² und R³, die gleich oder verschieden sind, Wasserstoffatom oder Alkyl mit 1 - 4 Kohlenstoffatomen bedeuten, und
R⁴ einen gesättigten Kohlenwasserstoffrest mit 6 bis 9 Kohlenstoffatomen oder einen Rest der Formel II bedeutet 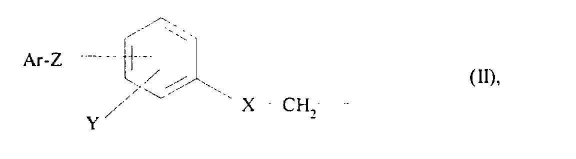 wobei
   - X: S oder O bedeutet,
   - Y: Wasserstoffatom oder bis zu 2 Halogenatome wie Chlor und/oder Brom bedeutet,
   - Z: eine Einfachbindung oder die zweiwertigen Reste O, S, -CR²-, worin R H oder C₁-C₄-Alkyl bedeutet, oder andere zweiwertige Reste mit 2- 10 kettenförmig verknüpften C- und gegebenenfalls O- und/oder S-Atomen, wobei - wenn die Reste 2 oder mehr O- und/oder S-Atome enthalten - letztere durch mindestens 2 C-Atome voneinander getrennt sein müssen und wobei 2 benachbarte C-Atome auch durch eine Doppelbindung miteinander verknüpft sein können und die freien Valenzen der C-Atome durch H und/oder C₁-C₄-Alkylgruppen abgesättigt sind, bedeutet,
   - Ar: ein aromatisches Ringsystem mit bis zu zwei Ringen, das durch bis zu drei Reste aus der Gruppe Fluor, Chlor, Brom, Methoxy, C₁-C₄-Alkyl,

Trifluormethyl und Trifluormethoxy substituiert sein kann, bedeutet zur Herstellung eines medizinischen Shampoo, enthaltend mindestens ein anionisches, kationisches, nichtionisches oder amphoteres Tensid oder eine Mischung der Tenside und das einen pH-Wert im hautphysiologischen Bereich aufweist zur Behandlung der Seborrhoischen Dermatitis.

In den Resten "Z" sind die C-Kettenglieder vorzugsweise CH₂-Gruppen. Wenn die CH₂-Gruppen durch C₁-C₄-Alkylgruppen substituiert sind, sind CH₃ und C₂H₅ bevorzugte Substituenten. Beispielhafte Reste "Z" sind: -O-, -S-, -CH₂-, -(CH₂)ₘ- (m = 2 - 10), -C(CH₃)₂-, -CH₂O-, -OCH₂-, -CH₂S-, -SCH₂-, -SCH(C₂H₅)-, -CH=CH-CH₂O-, -O-CH₂-CH=CH-CH₂O-, -OCH₂-CH₂O-, -OCH₂-CH₂CH₂O-, -SCH₂CH₂CH₂S-, -SCH₂CH₂CH₂CH₂O-, -SCH₂CH₂OCH₂CH₂O-, -SCH₂CH₂OCH₂CH₂O-CH₂CH₂S- oder -S-CH₂-C(CH₃)₂-CH₂-S-.

Der Rest "S" bedeutet Schwefelatom, der Rest "O" bedeutet Sauerstoffatom. Der Begriff "Ar" bedeutet Phenyl oder kondensierte Systeme wie Naphthyl, Tetrahydronaphthyl und Indenyl, sowie isolierte Systeme wie solche, die sich vom Biphenyl, Diphenylalkanen, Diphenylethern und Diphenylthioethern ableiten.

In der Formel I ist der Kohlenwasserstoff-Rest R⁴ ein Alkyl- oder Cyclohexylrest, der auch über eine Methylen- oder Äthylengruppe an den Pyridonring gebunden sein oder einen Endomethylgruppe enthalten kann. R⁴ kann auch einen aromatischen Rest darstellen, der jedoch vorzugsweise über wenigstens ein aliphatisches C-Atom an den Pyridonrest gebunden ist.

Wichtige Vertreter der durch die Formel I charakterisierten Verbindungsklasse sind:
6-[4-(4-Chlor-phenoxy)-phenoxymethyl]-1-hydroxy-4-methyl-2-pyridon, 6-[4-(2,4-Dichlor-phenoxy)-phenoxymethyl]-1-hydroxy-4-methyl-2-pyridon, 6-(Biphenylyl-4-oxy-methyl)-1-hydroxy-4-methyl-2-pyridon, 6-(4-Benzyl-phenoxymethyl)-1-hydroxy-4-methyl-2-pyridon, 6-[4-(2,4-Dichlorbenzyloxy)-phenoxy-methyl]-1-hydroxy-4-methyl-2-pyridon, 6-[4-(4-Chlor-phenoxy)-phenoxymethyl]-1-hydroxy-3,4-dimethyl-2-pyridon, 6-[4-(2,4-Dichlor-benzyl)-phenoxymethyl]-1-hydroxy-3,4-dimethyl-2-pyridon, 6-[4-(Cinnamyloxy)-phenoxymethyl]-1-hydroxy-4-methyl-2-pyridon, 1-Hydroxy-4-methyl-6-[4-(4-trifluormethyl-phenoxy)-phenoxymethyl]-2-pyridon, 1-Hydroxy-4-methyl-6-cyclohexyl-2-pyridon, 1-Hydroxy-4-methyl-6-(2,4,4-trimethylpentyl)-2-pyridon, 1-Hydroxy-4-methyl-6-n-hexyl-, -6-iso-hexyl-, -6-n-heptyl- oder -6-iso-heptyl-2-pyridon, 1-Hydroxy-4-methyl-6-octyl- oder -6-iso-octyl-2-pyridon, insbesondere 1-Hydroxy-4-methyl-6-cyclohexylmethyl- oder -6-cyclohexylethyl-2-pyridon, wobei der Cyclohexylrest jeweils auch einen Methylrest tragen kann, 1-Hydroxy-4-methyl-6-(2-bicyclo[2,2,1]heptyl)-2-pyridon, 1-Hydroxy-3,4-dimethyl-6-benzyl- oder -6-dimethylbenzyl-2-pyridon oder 1-Hydroxy-4-methyl-6-(β-phenyl-ethyl)-2-pyridon.

Der Begriff "gesättigt" bezeichnet hierbei solche Reste, die keine aliphatischen Mehrfachbindungen, also keine ethylenischen oder acetylenischen Bindungen enthalten.

Die obengenannten Verbindungen der Formel I können sowohl in freier Form als auch als Salze eingesetzt werden, die Verwendung in freier Form ist bevorzugt.

Kommen organische Basen zur Anwendung, so werden vorzugsweise schwere flüchtige Basen eingesetzt, beispielsweise niedrigmolekulare Alkanolamine wie Ethanolamin, Diethanolamin, N-Ethylethanolamin, N-Methyl-diethanolamin, Triäthanolamin, Diethylamino-ethanol, 2-Amino-2-methyl-n-propanol, Dimethylaminopropanol, 2-Amino-2-methyl-propandiol, Tri-isopropanolamin. Als weitere schwerer flüchtige Basen seien beispielsweise erwähnt Ethylendiamin, Hexamethylendiamin, Morpholin, Piperidin, Piperazin, Cyclohexylamin, Tributylamin, Dodecylamin, N,N-Dimethyl-dodecylamin, Stearylamin, Oleylamin, Benzylamin, Dibenzylamin, N-Ethylbenzylamin, Dimethylstearylamin, N-Methylmorpholin, N-Methylpiperazin, 4-Methylcyclohexylamin, N-Hydroxyethyl-morpholin. Auch die Salze quartärer Ammoniumhydroxide wie Trimethylbenzyl-ammonium-hydroxid, Tetramethylammoniumhydroxid oder Tetraethylammoniumhydroxid können verwendet werden, ferner Guanidin und seine Abkömmlinge, insbesondere seine Alkylierungsprodukte. Es ist jedoch auch möglich, als Salzbildner beispielsweise niedrigmolekulare Alkylamine wie Methylamin, Ethylamin oder Triethylamin einzusetzen. Auch Salze mit anorganischen Kationen, beispielsweise Alkalisalze, insbesondere Natrium-, Kalium- oder Ammonium-Salze, Erdalkalisalze wie insbesondere das Magnesium- oder Calciumsalz, sowie Salze mit zwei- bis vierwertigen Kationen, beispielsweise das Zink-, Aluminium- oder Zirkon-Salz kommen für die erfindungsgemäß einzusetzenden Verbindungen in Betracht.

Die in den Zubereitungen einzusetzenden Wirkstoffe der Verbindung der Formel I können beispielsweise nach Verfahren gemäß US 2 540 218 hergestellt werden.

Für den erfindungsgemäßen Einsatz der genannten Verbindungen kommen flüssige bis halbfeste pharmazeutische Zubereitungen in Betracht, insbesondere Haarwässer, Shampoos, flüssige Seifen, sowie Creme-, Salben- und Gelzubereitungen.

Es handelt sich dabei immer um Zubereitungen, die je nach ihrem eigentlichen Anwendungszweck für kürzere oder längere Zeit auf die Haut und/oder auf die Kopfhaut aufgebracht werden. Durch die Zugabe der erfindungsgemäß verwendeten Verbindungen wird eine effektive Behandlung der Seborrhoischen Dermatitis bewirkt.

Liegen die erfindungsgemäß verwendeten Zubereitungen als Shampoo vor, so können sie klarflüssig, opakflüssig, cremeförmig oder auch gelartig sein. Die diesen Shampoos zugrunde liegenden Tenside können anionischer, kationischer, nichtionischer und amphoterer Natur sein und auch in Kombination dieser Stoffe vorliegen.

Bevorzugt werden jedoch anionische Tenside alleine oder in Mischung mit anderen anionischen Tensiden als Basistenside - gegebenenfalls unter Zusatz von amphoteren Tensiden als Cotensid - eingesetzt.

Amphotere Tenside sind als alleinige waschaktive Substanzen praktisch unbedeutend, da bei ihnen Schaumverhalten, Verdickbarkeit und teils auch Haut- und Augenschleimverträglichkeit nur mäßig sind. In Kombination mit verschiedenen anionischen Tensiden werden aber gerade diese Eigenschaften synergistisch verbessert. Dies erklärt die relativ große Bedeutung der amphoteren Tenside zur Optimierung von anionischen Shampoo Grundlagen.

Ebenfalls können nichtionogene Tenside als Cotenside eingesetzt werden.

Als Beispiel für derartige anionische waschaktive Substanzen seien genannt:
(C₁₀-C₂₀₎-Alkyl- und Alkylen-carboxylate, Alkylethercarboxylate, Fettalkoholsulfate, Fettalkoholethersulfate, Alkylolamidsulfate und -sulfonate, Fettsäurealkyolamidpolyglykolethersulfate, Alkansulfonate und Hydroxyalkansulfonate. Olefinsulfonate, Acylester von Isothionaten, α-Sulfofettsäureester, Alkylbenzosulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate oder Sulforicinoleate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammoniumsowie analogen Alkylolammonium-Salze.

Beispiele für den Shampoos zusetzbare Amphotenside sind: N-((C₁₂-C₁₈₎-Alkyl)-β-aminopropionate und N-((C₁₂-C₁₈)-Alkyl)-β-iminodipropionate als Alkali- und Mono-, Di- und Trialkylolammonium-Salze; N-Acylamidoalkyl-N,N-dimethyl-acetobetain, vorzugsweise N-((C₈-C₁₈₎-Acyl)amidopropyl-N,N-dimethyl-acetobetain; (C₁₂-C₁₈₎-Alkyldimethylsulfopropylbetain; Amphotenside auf Basis Imidazolin (Handelsname: Miranol®, Steinapon®), vorzugsweise das Natrium-Salz des 1-(β-Carboxymethyloxyethyl)-1-(carboxymethyl)-2-lauryl-imidazoliniums; Aminoxide, z. B. (C₁₂-C₁₈₎- Alkyldimethylaminoxid oder Fettsäureamidoalkyldimethylaminoxid.

Als nicht ionogene Tenside, die als waschaktive Substanzen eingesetzt werden können, kommen beispielsweise in Betracht: Fettalkoholethoxylate (Alkylpolyethylenglykole); Alkylphenolpolyethylenglykole; Alkylmercaptanpolyethylenglykole; Fettaminethoxylate (Alkylaminpolyethylenglykole); Fettsäureethoxylate (Acylpolyethylenglykole), Polypropylenglykolethoxylate (Pluronic®); Fettsäurealkylolamide (Fettsäureamidpolyethylenglykole); Saccharoseester; Alkylpolyglucoside; Sorbitester und der Polyglykolether.

Geeignete kationische Tenside sind beispielsweise quartäre Ammonium-Salze wie Di-((C₁₀-C₂₄)-Alkyl)-dimethyl-ammonium-chlorid oder -bromid, vorzugsweise Di-((C₁₂-C₁₈)-Alkyl)-dimethyl-ammonium-chlorid oder - bromid; (C₁₀-C₂₄)-Akyldimethylethylammonium-chlorid oder -bromid; (C₁₀-C₂₄)-Alkyltrimethylammoniumchlorid oder -bromid, vorzugsweise Cetyltrimethylammonium-chlorid oder -bromid und (C₂₀-C₂₂)-Alkyltrimethylammonium-chlorid oder -bromid; (C₁₀-C₂₄)-Alkyldimethylbenzylammonium-chlorid oder -bromid, vorzugsweise (C₁₂-C₁₈)-Alkyldimethylbenzylammoniumchlorid; N-((C₁₀-C₁₈)-Alkyl)-pyridinium-chlorid oder - bromid, vorzugsweise N-((C₁₂-C₁₆)-Alkyl)pyridinium-chlorid oder -bromid; N-((C₁₀-C₁₈)-Alkyl)isochinolinium-chlorid, -bromid oder -monoalkylsulfat; N-((C₁₂-C₁₈)-Alkylcolaminoformylmethyl)pyridinium-chlorid; N-((C₁₂-C₁₈)-Alkyl)-N-methylmorpholinium-chlorid, -bromid oder -monoalkylsulfat, N-((C₁₂-C₁₈)-Alkyl)-N-ethylmorpholinium-chlorid, -bromid oder-monoalkylsulfat; (C₁₆-C₁₈)-Alkyl-pentaoxethylammoniumchlorid; Di-isobutylphenoxyethoxyethyldimethylbenzylammoniumchlorid; Salze des N,N-Diethylaminoethylstearyl-amids- und -oleylamids mit Salzsäure, Essigsäure, Milchsäure, Zitronensäure, Phosphorsäure; N-Acylamidoethyl-N,N-diethyl-N-methyl-ammoniumchlorid, -bromid oder -monoalkylsulfat und N-Acylamido-ethyl-N,N-diethyl-N-benzyl-ammonium-chlorid, -bromid oder - monoalkylsulfat, wobei Acyl vorzugsweise für Stearyl oder Oleyl steht.

Die erfindungsgemäß verwendeten Zubereitungen können außerdem weitere Zusätze enthalten, z. B. Riechstoffe, Farbstoffe, Trübungsmittel und Perlglanzmittel, beispielsweise Ester von Fettsäuren und Polyolen, Magnesium- und Zinksalze von Fettsäuren, Dispersionen auf Basis Mischpolymerer, Verdickungsmittel wie Natrium-, Kalium-, Ammoniumchlorid, Natriumsulfat, Fettsäurealkylolamide, Cellulosederivate natürliche Gummen, Collagenhydrolysate, ferner Fette, Öle, Fettalkohole, Silikone, Stoffe mit keratolytischer und keratoplastischer Wirkung, beispielweise Schwefel, Salicylsäure oder Enzyme.

Die Herstellung der Shampoos erfolgt in an sich bekannter Weise durch Zusammengeben der einzelnen Komponenten und eine - soweit erforderlich - der jeweiligen Zubereitungsart angepaßte Weiterverarbeitung. Einige dieser vielfältigen möglichen Zubereitungen werden in den Ausführungsbeispielen beispielhaft beschrieben.

Die erfindungsgemäß verwendeten Zubereitungen können auch in Form von wäßrigen und wäßrig-alkoholischen Haarwässern, auch solcher in Gelform und in Aerosolform als Spray oder Schaum vorliegen. Als Alkohol werden vorzugsweise Ethanol und Isopropylalkohol eingesetzt.

Als weitere Zubereitungen, in denen die 1-Hydroxy-2-pyridone erfindungsgemäß zur Anwendung kommen können, seien beispielsweise Creme- und Salbenzubereitungen genannt, Produkte, die in erster Linie zur Behandlung von haarlosen Kopf- und Körperpartien Verwendung finden.

Auch die Herstellung aller dieser Zubereitungen erfolgt - wie bereits beim Shampoo erwähnt - in an sich bekannter Weise unter Zugabe des erfindungsgemäß eingesetzten Wirkstoffs. Die erfindungsgemäß verwendeten Zubereitungen können von den oben genannten 1-Hydroxy-2-pyridonen eine Verbindung oder auch mehrere in Kombination enthalten.

Der pH-Wert der Zubereitungen liegt im hautphysiologischen Bereich von etwa pH 4,5 bis 6,5. Während bei Verwendung der Verbindungen in Salzform die Einstellung des genannten pH Bereiches mit organischen Säuren erfolgen muß, ist diese Maßnahme bei Verwendung der freien Verbindungen nicht erforderlich.

In den erfindungsgemäß verwendeten Zubereitungen wird der Wirkstoff in Mengen eingearbeitet, die üblicherweise zwischen etwa 0,05 und etwa 10 % liegen. Innerhalb dieses Bereiches richten sich die Konzentrationen der speziellen Zubereitungen nach ihrem Anwendungszweck. Bestimmte Zubereitungsformen wie Konzentrate, die vor ihrer Anwendung zu verdünnen sind, können erheblich höhere Konzentrationen aufweisen.

Handelt es sich um Zubereitungen, die auf der Haut und auf der Kopfhaut verbleiben, beispielsweise Gelzubereitungen, Salben, Cremes oder Haarwässer, so wird man niedrigere Konzentrationen einsetzen, beispielsweise von ca. 0,05 % bis ca. 1%, vorzugsweise von 0,1 bis 0,5 %. In höheren Konzentrationen werden sie zweckmäßigerweise dann zur Anwendung kommen, wenn es sich um Zubereitungen handelt, die, gegebenfalls nach Verdünnung, nur kurze Zeit auf die Kopfhaut einwirken, beispielsweise Shampoos oder flüssige Seifen. In diesen Fällen können z. B. Konzentrationen von etwa 0,2 bis etwa 10 %, vorzugsweise von etwa 0,5 % bis etwa 2 %, zweckmäßig sein.

Die nachfolgenden Mengenangaben beziehen sich auf das Gewicht, soweit es nicht anders vermerkt ist.

### Beispiel 1

Eine erfindungsgemäß verwendete Zubereitung weist folgende Zusammensetzung auf:

### Shampoo

### (auf Basis anionischer waschaktiver Substanzen)

| | |
|---|---|
| 1-Hydroxy-4-methyl-6-cyclohexyl-2(1H)-pyridon | 1,00 % |
| Natriumlauryldiglykolethersulfat (27%ige Lösung) | 40,00 % |
| Dinatriumlaurylpolyglykolethersulfosuccinat (33%ige Lösung) | 10,00 % |
| Natriumchlorid | 2,50 % |
| Wasser | 46,50 % |

### Beispiel 2

Eine erfindungsgemäß verwendete Zubereitung weist folgende Zusammensetzung auf:

### Shampoo

### (auf Basis anionischer waschaktiver Substanz mit Amphotensid als Cotensid)

| | |
|---|---|
| 1-Hydroxy-4-methyl-6-cyclohexyl-2(1H)-pyridon | 1,00 % |
| Natriumlauryldiglykolethersulfat (27%ige Lösung) | 36,00 % |
| Cocamidopropylbetain (30%ige Lösung) | 6,00 % |
| Natriumchlorid | 3,30 % |
| Wasser | 53,70 % |

### Beispiel 3

Eine erfindungsgemäß verwendete Zubereitung weist folgende Zusammensetzung auf:

### Shampoo

### (auf Basis anionischer waschaktiver Substanz mit nichtionogenem Tensid als Cotensid)

| | |
|---|---|
| 1-Hydroxy-4-methyl-6-cyclohexyl-2(1H)-pyridon | 1,50 % |
| Natriumlauryldiglykolethersulfat (27%ige Lösung) | 30,00 % |
| Laurylalkoholpolyglukosid | 8,00 % |
| Natriumchlorid | 2,00 % |
| Wasser | 58,50 % |

### Beispiel 4

Eine erfindungsgemäß verwendete Zubereitung weist folgende Zusammensetzung auf:

### Flüssige Seife

| | |
|---|---|
| 1-Hydroxy-4-methyl-6-cyclohexyl-2(1H)-pyridon | 1,00 % |
| Natriumlauryldiglykolethersulfat (27%ige Lösung) | 35,00 % |
| Cocamidopolyglykolether Sulfat-Magnesiumsalz (30%ige Lösung) | 8,00 % |
| Cocamidopropylbetain (30%ige Lösung) | 10,00 % |
| Laurylalkoholglykolether | 2,00 % |
| Natriumchiorid | 2,00 % |
| Wasser | 42,00 % |

### Beispiel 5

Eine erfindungsgemäß verwendete Zubereitung weist folgende Zusammensetzung auf:

### Haarwasser

| | |
|---|---|
| 1-Hydroxy-4-methyl-6-[4(4-chlorphenoxy)phenoxymethyl]-2(1H)pyridon | 0,05 % |
| 2-Propanol | 60,00 % |
| Wasser | 39,95 % |

### Beispiel 6

Eine erfindungsgemäß verwendete Zubereitung weist folgende Zusammensetzung auf:

### Gelzubereitung

| | |
|---|---|
| 1-Hydroxy-4-methyl-cyclohexyl-2(1H)pyridon | 0,75 % |
| 2-Propanol | 15,00 % |
| 2-Octyldodecanol | 7,50 % |
| Carbomer 4 000 000 | 0,50 % |
| Polysorbat 60 | 1,50 % |
| Natriumhydroxid | 0,18 % |
| Wasser | 74,57 % |

### Beispiel 7

Eine erfindungsgemäß verwendete Zubereitung weist folgende Zusammensetzung auf:

### Cremezubereitung

| | |
|---|---|
| 1-Hydroxy-4-methyl-6-cyclohexyl-2(1H)pyridon, Aminoethanolsalz 1:1 | 1,00 % |
| 2-Octyldodecanol | 7,50 % |
| Paraffinöl | 7,50 % |
| Stearylalkohol | 7,50 % |
| Cetylalkohol | 7,50 % |
| Polysorbat 60 | 3,00 % |
| Sorbitanmonostearat | 2,00 % |
| Milchsäure 90%ig | 0,51 % |
| Wasser | 63,49 % |

### Beispiel 8

In einer klinischen Studie mit insgesamt 180 Patienten konnte gezeigt werden, daß die Symptome einer Seborrhoischen Dermatitis der Kopfhaut (schwere Schuppung, Entzündung, Juckreiz) durch eine 1 - 2 x wöchentliche Behandlung mit einer 1 %igen Ciclopirox Shampoozubereitung über einen Zeitraum von 4 Wochen hinweg wirksam therapiert werden können.

### Beispiel 9

In einer klinischen Studie konnten insgesamt 180 Patienten mit einer Seborrhoischen Dermatitis der Kopfhaut, des Gesichtes und des Oberkörpers erfolgreich durch Applikation einer 0,77 %igen Ciclopirox Gelzubereitung über einen Zeitraum von 4 Wochen behandelt werden.

## Patentansprüche

1. Die Verwendung von 1-Hydroxy-2-pyridon der Formel I 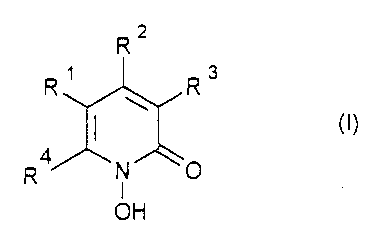 worin
R¹, R² und R³, die gleich oder verschieden sind, Wasserstoffatom oder Alkyl mit 1 - 4 Kohlenstoffatomen bedeuten, und
R⁴ einen gesättigten Kohlenwasserstoffrest mit 6 bis 9 Kohlenstoffatomen oder einen Rest der Formel II bedeutet 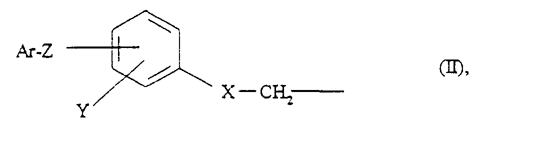 wobei
X S oder O bedeutet,
Y Wasserstoffatom oder bis zu 2 Halogenatome wie Chlor und/oder Brom bedeutet,
Z eine Einfachbindung oder die zweiwertigen Reste O, S, -CR²-, worin R H oder C₁-C₄-Alkyl bedeutet, oder andere zweiwertige Reste mit 2 - 10 kettenförmig verknüpften C- und gegebenenfalls O- und/oder S-Atomen, wobei - wenn die Reste 2 oder mehr O- und/oder S-Atome enthalten - letztere durch mindestens 2 C-Atome voneinander getrennt sein müssen und wobei 2 benachbarte C-Atome auch durch eine Doppelbindung miteinander verknüpft sein können und die freien Valenzen der C-Atome durch H und/oder C₁-C₄-Alkylgruppen abgesättigt sind, bedeutet,
Ar ein aromatisches Ringsystem mit bis zu zwei Ringen, das durch bis zu drei Reste aus der Gruppe Fluor, Chlor, Brom, Methoxy, C₁-C₄-Alkyl, Trifluormethyl und Trifluormethoxy substituiert sein kann, bedeutet,
zur Herstellung eines medizinischen Shampoo, enthaltend mindestens ein anionisches, kationisches, nichtionisches oder amphoteres Tensid oder eine Mischung der Tenside und das einen pH-Wert im hautphysiologischen Bereich aufweist zur Behandlung der Seborrhoischen Dermatitis.

2. Verwendung gemäß Anspruch 1, **dadurch gekennzeichnet, daß** man die Verbindung der Formel I einsetzt, worin Ar ein bicyclisches System darstellt, das sich vom Biphenyl, Diphenylalkan oder Diphenylether ableitet.

3. Verwendung gemäß Anspruch 1 oder 2, **dadurch gekennzeichnet, daß** die Verbindung der Formel I in der Position R⁴ einen Cyclohexylrest enthält.

4. Verwendung gemäß einem oder mehreren der Ansprüche 1 bis 3, **dadurch gekennzeichnet, daß** die Verbindung der Formel I in der Position R⁴ einen Octylrest der Formel -CH₂-CH(CH₃)-CH₂-C(CH₃)₃ enthält.

5. Verwendung gemäß Anspruch 1, **dadurch gekennzeichnet, daß** man 1-Hydroxy-4-methyl-6-[4-(4-chlorphenoxy)-phenoxymethyl]-2-(1H)pyridon, 1-Hydroxy-4-methyl-6-cyclohexyl-2-(1 H)pyridon oder 1-Hydroxy-4-methyl-6-(2,4,4-trimethylpentyl)-2-(1 H)pyridon einsetzt.

6. Verwendung gemäß Anspruch 1, **dadurch gekennzeichnet, daß** man als Tensid mindestens ein anionisches Tensid allein oder in Mischung mit anderen anionischen Tensiden und/oder amphoteren Tensiden einsetzt.

7. Verwendung nach Anspruch 1, **dadurch gekennzeichnet, daß** man das 1-Hydroxy-2-pyridon der Formel I in einer Konzentration von 0,2 % bis 10 einsetzt.

8. Verwendung nach Anspruch 7, **dadurch gekennzeichnet**, das man das 1-Hydroxy-2-pyridon der Formel I in einer Konzentration von 0,5 % bis 2 % einsetzt.

9. Verwendung nach Anspruch 1, **dadurch gekennzeichnet, daß** der medizinische Shampoo einen pH-Wert von etwa 4,5 bis 6,5 aufweist.

## Claims

1. The use of 1-hydroxy-2-pyridones of the formula I 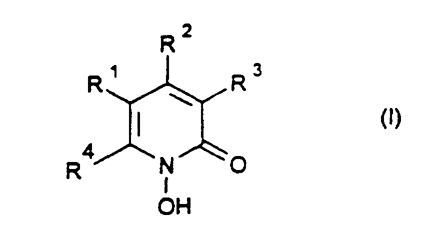 in which
R¹, R² and R³, which are identical or different, are a hydrogen atom or alkyl having 1-4 carbon atoms, and
R⁴ is a saturated hydrocarbon radical having 6 to 9 carbon atoms or a radical of the formula II 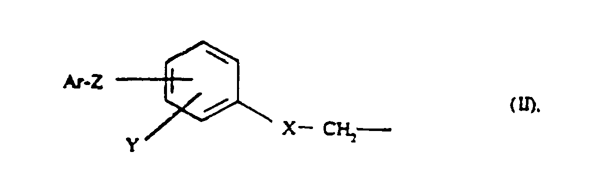 where
X is S or O,
Y is a hydrogen atom or up to 2 halogen atoms such as chlorine and/or bromine,
Z is a single bond or the bivalent radicals O, S, -CR²-, in which R is H or C₁-C₄-alkyl, or other bivalent radicals having 2-10 carbon and, if appropriate, oxygen and/or sulfur atoms linked in the form of a chain, where - if the radicals contain 2 or more oxygen and/or sulfur atoms - the latter must be separated from one another by at least 2 carbon atoms and where 2 adjacent carbon atoms can also be linked to one another by a double bond and the free valences of the carbon atoms are saturated by H and/or C₁-C₄-alkyl groups,
Ar is an aromatic ring system having up to two rings which can be substituted by up to three radicals from the group consisting of fluorine, chlorine, bromine, methoxy, C₁-C₄-alkyl, trifluoromethyl and trifluoromethoxy,
for the production of a medicinal shampoo comprising at least one anionic, cationic, nonionic or amphoteric surfactant or a mixture of the surfactants and which has a pH in the dermatophysiological range for the treatment of seborrheic dermatitis.

2. The use as claimed in claim 1, wherein the compound of the formula I is employed in which Ar is a bicyclic system which is derived from biphenyl, diphenylalkane or diphenyl ether.

3. The use as claimed in claim 1 or 2, wherein the compound of the formula I contains a cyclohexyl radical in the position R⁴.

4. The use as claimed in one or more of claims 1 to 3, wherein the compound of the formula I contains an octyl radical of the formula -CH₂-CH(CH₃)-CH₂-C(CH₃)₃ in the position R⁴.

5. The use as claimed in claim 1, wherein 1-hydroxy-4-methyl-6-[4-(4-chlorophenoxy)phenoxymethyl]-2(1H)pyridone, 1-hydroxy-4-methyl-6-cyclohexyl-2(1H)pyridone or 1-hydroxy-4-methyl-6-(2,4,4-trimethylpentyl)-2(1H)pyridone is employed.

6. The use as claimed in claim 1, wherein the surfactant employed is at least one anionic surfactant on its own or as a mixture with other anionic surfactants and/or amphoteric surfactants.

7. The use as claimed in claim 1, wherein the 1-hydroxy-2-pyridone of the formula I is employed in a concentration of 0.2% to 10%.

8. The use as claimed in claim 7, wherein the 1-hydroxy-2-pyridone of the formula I is employed in a concentration of 0.5% to 2%.

9. The use as claimed in claim 1, wherein the medicinal shampoo has a pH of approximately 4.5 to 6.5.

## Revendications

1. Utilisation de 1-hydroxy-2-pyridone de formule I, 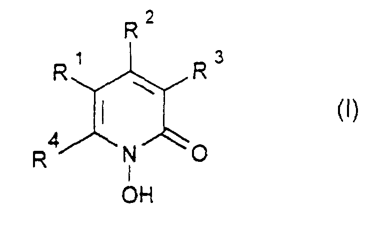 dans laquelle
R¹, R² et R³, qui sont identiques ou différents, représentent un atome d'hydrogène ou un groupe alkyle ayant de 1 à 4 atomes de carbone, et
R⁴ représente un groupe hydrocarboné insaturé ayant de 6 à 9 atomes de carbone ou un groupe de formule II 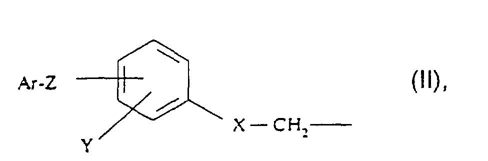 dans laquelle
X représente S ou O,
Y représente un atome d'hydrogène ou jusqu'à 2 atomes d'halogène comme le chlore et/ou le brome,
Z représente une liaison simple ou les groupes bivalents O, S, -CR²-, dans lesquels R représente H ou un groupe alkyle en C₁ à C₄ ou d'autres groupes bivalents ayant de 2 à 10 atomes de carbone et éventuellement 0 et/ou S liés en forme de chaîne, dans lesquels, lorsque les groupes contiennent 2 atomes O et/ou S ou plus, ces derniers doivent être séparés l'un de l'autre par au moins 2 atomes de carbone et dans lesquels 2 atomes de carbone voisins peuvent être également reliés entre eux par une double liaison et les valences libres des atomes de carbone sont saturées par H et/ou des groupes alkyle en C₁ à C₄,
Ar représente une combinaison cyclique aromatique ayant jusqu'à deux cycles, qui peut être substituée par jusqu'à trois groupes choisis parmi le fluor, le chrome, le brome, le méthoxy, un groupe alkyle en C₁ à C₄, le trifluorométhyle et le trifluorométhoxy,
pour la préparation d'un shampoing médicinal, contenant au moins un agent tensio-actif anionique, cationique, non ionique ou amphotère ou un mélange des tensio-actifs et qui présente une valeur de pH dans le domaine physiologique de la peau pour le traitement de l'eczéma séborrhéïque.

2. Utilisation selon la revendication 1, **caractérisée en ce que** l'on utilise le composé de formule I, dans lequel Ar représente un système bicyclique, qui dérive du biphényl, du diphénylalcane ou du diphényléther.

3. Utilisation selon la revendication 1 ou 2, **caractérisée en ce que** le composé de formule I contient en position R⁴ un groupe cyclohexyle.

4. Utilisation selon une ou plusieurs des revendications 1 à 3, **caractérisée en ce que** le composé de formule I contient en position R⁴ un groupe octyle de formule -CH₂-CH(CH₃)-CH₂-C(CH₃)₃.

5. Utilisation selon la revendication 1, **caractérisée en ce que** l'on utilise la 1-hydroxy-4-méthyl-6-[4-(4-chlorophénoxy)-phénoxyméthyl]-2-(1H)-pyridone, la 1-hydroxy-4-méthyl-6-cyclohexyl-2-(1H)-pyridone ou la 1-hydroxy-4-méthyl-6-(2,4,4-triméthylpentyl)-2-(1H)-pyridone.

6. Utilisation selon la revendication 1, **caractérisée en ce qu'**on utilise comme agent tensio-actif au moins un agent tensio-actif anionique seul ou en mélange avec d'autres agents tensio-actifs anioniques et/ou tensio-actifs amphotères.

7. Utilisation selon la revendication 1, **caractérisée en ce qu'**on utilise la 1-hydroxy-2-pyridone de formule I en une concentration de 0,2 % à 10 %.

8. Utilisation selon la revendication 7, **caractérisée en ce qu'**on utilise la 1-hydroxy-2-pyridone de formule I en une concentration de 0,5 % à 2 %.

9. Utilisation selon la revendication 1, **caractérisée en ce que** le shampoing médicinal présente une valeur de pH d'environ 4,5 % à 6,5 %.
